# EUROPEAN PATENT SPECIFICATION

(11) **EP 3 082 865 B1**
(45) Date of publication and mention of the grant of the patent: **11.08.2021**
(21) Application number: 14816271.2
(22) Date of filing: 19.12.2014
(51) Int. Cl.: A61K 31/122, A61K 31/202, A61K 31/455, A61K 31/592, A61K 31/616, A61K 31/714, A61K 45/06, A61P 7/02, A61P 9/00, A23K 20/158, A23K 20/174, A23L 33/12, A23L 33/15

(54) **PREVENTION AND COUNTERACTION OF DIET-INDUCED THROMBOSIS RISK**
PRÄVENTION UND GEGENAKTION VON DIÄTINDUZIERTER THROMBOSEGEFAHR
PRÉVENTION ET ACTION CONTRAIRE DE RISQUE DE THROMBOSE INDUITE PAR LE RÉGIME ALIMENTAIRE

(30) Priority: 20.12.2013 EP 13198969
(43) Date of publication of application: 26.10.2016
(73) Proprietor: NattoPharma ASA, 0283 Oslo (NO)
(72) Inventor: VERMEER, Cees, 6229 EV Maastricht (NL)
(74) Representative: Acapo AS
(86) International application number: PCT/EP2014/078866
(87) International publication number: WO 2015/092018

(56) References cited:
- WO-A1-2012/161572
- WO-A2-2008/006607
- WO-A2-2009/063485
- US-A1- 2005 261 257
- RONDEN J E ET AL: "Modulation of arterial thrombosis tendency in rats by vitamin K and its side chains.", ATHEROSCLEROSIS 11 JUL 1997, vol. 132, no. 1, 11 July 1997 (1997-07-11) , pages 61-67, XP002723037, ISSN: 0021-9150
- PINI MARIO ET AL: "Prevention of venousthromboembolism", SEMINARS IN THROMBOSIS AND HEMOSTASIS, THIEME MEDICAL PUBLISHERS, INC, US, vol. 32, no. 8 31 October 2006 (2006-10-31), pages 755-766, XP009525823, ISSN: 0094-6176, DOI: 10.1055/S-2006-955458 [retrieved on 2006-12-15]

## Description

### FIELD OF THE INVENTION

This invention relates to the field of *nutrition* and the influence of dietary vitamin K content on the complex interactions between procoagulant and anticoagulant factors resulting in the controlled process of *haemostasis.* Even minor impairments in the synthesis or activity of one of the individual components in this cascade may result in an increased risk for either prolonged bleeding or a thrombosis tendency. This invention describes the preparation of nutraceutical and pharmaceutical products which may counteract or prevent an increased venous thrombosis tendency.

The use of vitamin K is provided in preparing pharmaceutical or nutraceutical products for counteracting an increased venous thrombosis risk caused by poor vitamin K status. The most likely mechanism underlying the vitamin K-insufficiency-induced thrombosis risk is undercarboxylation of vascular protein S. Preferably, vitamin K is a menaquinone, most preferably selected from the long-chain menaquinones MK-7 through MK-10.

### BACKGROUND OF THE INVENTION

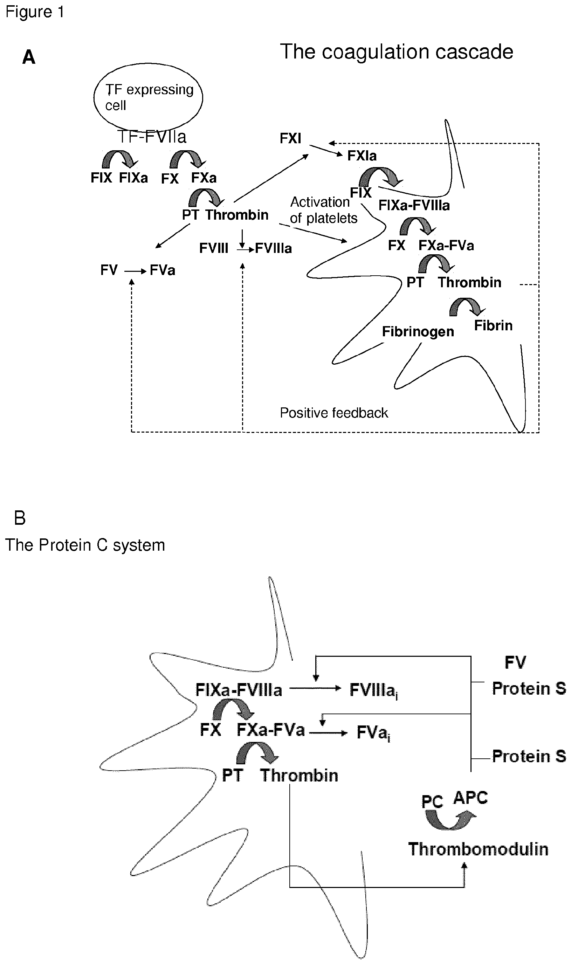

Blood coagulation is a complex process which can be initiated either via the intrinsic pathway or via the extrinsic pathway (see Figure 1A). Activation of the various coagulation factors is accomplished by proteolytic cleavage by which they are converted into either another proteolytic enzyme (e.g.: factors X, IX, VII and II) or an activated structural protein (factors VIII, V, and fibrinogen). In the intrinsic pathway coagulation factor XII is surface-activated to subsequently activate factors XI, IX and X in this cascade. In the extrinsic pathway, tissue factor (TF) activates factor VII which further activates factor X. These processes take place at phospholipid surfaces and are accelerated by their tight binding to the phospholipid and a structural clotting factor (B. Dahlbäck, B.O Villoutreix: Arterioscler. Thromb. Vasc. Biol. 2005;25:1311-1320). For the conversion of factor X into activated factor X (factor Xa) the structural clotting factor is factor VIIIa, and the proteolytic enzyme is factor IXa. For the conversion of factor II (prothrombin) into factor IIa (thrombin) the structural clotting factor is factor Va , and the proteolytic enzyme is factor Xa. Thrombin is the key enzyme, degrading fibrinogen into fibrin monomers which rapidly aggregate into a complex fibrin network generally known as a blood clot. Four of the various coagulation factors belong to the family of the "Gla-proteins", which means that they contain the unusual amino acid gammacarboxyglutamate (Gla): prothrombin (also known as coagulation factor II), factor VII, factor IX and factor X (C. Vermeer, E. Theuwissen: Menopause Int. 2011;17:19-23*).* Gla-residues in these proteins are formed by the action of vitamin K in a posttranslational carboxylation step by which a number of well-identified glutamate residues are converted into Gla. The Gla-residues form calcium-binding groups in these proteins. In case calcium ions are bound the proteins undergo a conformational change (as is the case with the clotting factors), but Gla-residues may also facilitate binding of the Gla-protein to calcium salt crystals (e.g. in osteocalcin and matrix Gla-protein). At this time 17 Gla-proteins are known, and in all cases the Gla-residues are essential for their biological activity. All four vitamin K-dependent clotting factors are exclusively synthesized in the liver. As early as in 1929 vitamin K was discovered because of its procoagulant activity (the character K stems from the German word Koagulation) and until now vitamin K has been generally regarded as a vitamin that promotes blood coagulation.

Besides the four Gla-proteins with procoagulant activity, also three Gla-proteins with anti-coagulant activity are known: proteins C, S, and Z. Whereas the function of protein Z has remained obscure, the vital importance of proteins C and S has become clear during recent years. Protein C is activated by thrombin cleavage into activated protein C (APC); thrombomodulin acts as a cofactor in this step. APC, on its turn, inactivates the clotting factors Va and VIIIa and these steps are greatly enhanced by protein S which acts as a cofactor for APC. Whereas protein C is exclusively synthesized in the liver, protein S is synthesized for 50% in the liver and 50% in the blood vessels. The delicate balance between the procoagulant and anticoagulant systems is of vital importance for survival: a slight deviation may result in either increased thrombosis risk or bleeding. Thrombotic events generally originate in the veins, and the local production of protein S may be a key factor in preventing thrombosis in an early stage. Importantly, it has been found that matrix Gla-protein (MGP), another Gla-protein of vascular origin, is incompletely carboxylated (E. Theuwissen, E. Smit, C. Vermeer: Adv. Nutr. 2012;3:166-173*),* i.e. in the normal, adult population between 20 and 30% of the circulating MGP occurs in the uncarboxylated form (ucMGP). This demonstrates that - in contrast to the liver - vitamin K status of vascular tissue is insufficient in the majority of the population. A similar observation was made for bone (undercarboxylation of osteocalcin), suggesting a more general pharmacokinetic mechanism by which vitamin K-insufficiency of extra-hepatic tissues is common in the healthy, Western population (C. Vermeer, M.J. Shearer, A. Zittermann, C. Bolton-Smith, P. Szulc, S. Hodges, P. Walter, W. Rambeck, E. Stöcklin, P. Weber. Eur. J. Nutr. 2004;43:1-11*).* In general there is a tight correlation between osteocalcin and MGP undercarboxylation. MGP is a potent inhibitor of vascular calcification, and its full carboxylation is essential for maximal calcification inhibitory activity (Schurgers, L.J., Cranenburg, E., Vermeer, C. Thromb. Haemostas. 2008;100:593-596). PINI MARIO ET AL: "Prevention of venous thromboembolism", SEMINARS IN THROMBOSIS AND HEMOSTASIS NOV 2006; 32:755-766, discloses low-dose unfractionated heparin or low molecular weight heparins (LMWHs), fondaparinux, or vitamin K antagonist for the prevention of venous thromboembolism. It has now surprisingly been found that due to vitamin K insufficiency, the protein S system is not fully active and that this defect can be remedied by administration of vitamin K.

In the context of the present invention, it is important to note that one has to differentiate between venous and arterial thrombosis.

Arterial thrombosis is the formation of a thrombus within an artery, generally as a result of rupture of an atherosclerotic plaque. This creates a wound and a foreign surface which form the trigger for thrombus formation in the artery, often referred to as atherothrombosis. In the animal model described by Ronden et al. (Ronden, J.E., Groenen-van Dooren, M.M.C.L., Hornstra, G., Vermeer, C. Modulation of arterial thrombosis tendency in rats by vitamin K and its side chains. Atherosclerosis 132 (1997) 61-67) the vascular damage and foreign surface are mimicked by inserting a plastic loupe in the aorta, so this is the most clear cut example of arterial thrombosis. Blood clots that are transported are usually entrapped in the coronary arteries or the brain. Therefore, the most common clinical manifestations seen as a consequence of arterial thrombosis are myocardial infarction (caused by a blockade of one of the coronary arteries), and infarction of the brain (caused by a clot formed in the carotid artery, eventually transported further into the brain).

Venous thrombosis is the formation of a blood clot (thrombus) in the veins. A common type of venous thrombosis is deep vein thrombosis (DVT), which is a blood clot in the deep veins of the leg. If the thrombus breaks off and flows upwards to the lungs, it is entrapped in the smaller vessels in the lungs and can become a life threatening pulmonary embolism. The underlying mechanism of venous thrombosis is often chronic activation of the clotting system (e.g. by atrial fibrillation), decreased inactivation of clotting factors (e.g. in Factor V_{Leiden}) or impaired blood flow (e.g. during long-distance air flights or immobilization). Unlike in arterial thrombosis, blood clots transported are entrapped in the lungs where they cause pulmonary embolism.

The present invention is particularly concerned with the prevention of venous thrombosis risk by increased vitamin K intake. Thus, any mention of "thrombosis risk" herein refers in particular to "venous thrombosis risk". Vitamin K is a group name for a family of related compounds generally classified as phylloquinone (vitamin K1) and menaquinones (vitamin K2).

Vitamin K1 (phylloquinone) consists of a methylated naphthoquinone ring structure, at the 3-position conjugated with a side chain composed of 4 isoprenoid units one of which is unsaturated. Phylloquinone is of plant origin and nutritionally relevant sources are green leafy vegetables. Whereas phylloquinone is one single compound, the menaquinones form a group of compounds having the methylated naphthoquinone ring in common, but differing with respect to the length and the degree of saturation of the polyisoprenoid side chain.

Vitamin K2 is a group of compounds called menaquinones ("MK") which are all 2-methyl-3-all-trans-polyprenylated-1,4-naphthoquinones having the following structural formula:

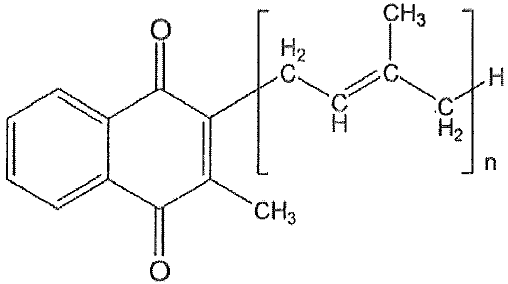

According to the generally used nomenclature, menaquinones are denoted as MK-n, where n gives the number of isoprenoid residues all of which are unsaturated. The various MKs are normally referred to as MK-2, MK-3, MK-4, MK-6 and so on. The number refers to the number of isoprene units (n=2, n=3, n=4, n=6, ...). So MK-8 (menaquinone-8) stands for vitamin K2 having a side chain comprising 8 isoprenoid residues and 8 double bonds in that chain.

The term vitamin K2 is a group name referring to all menaquinones of the MK-n structure. Natural forms of vitamin K2 found in the human diet include MK-4 through MK-13, shorter or longer menaquinones have been reported but are rarely found in food. Menaquinones are of bacterial origin and are mainly found in fermented foods such as cheese, curd cheese, sauerkraut and the Japanese food natto (fermented soy beans) (M.J. Shearer, X. Fu, S.L. Booth: Adv. Nutr. 2012;3:182-195*).*

The different aliphatic side chain in menaquinones results in different pharmacokinetics as compared to phylloquinone, resulting in differential transport systems. As a consequence, phylloquinone is preferentially transported to the liver, whereas K2 has more importance for extra-hepatic tissues including bone and vessel wall (L.J. Schurgers, C. Vermeer: Biochim. Biophys. Acta 2002;1570: 27-32*).*

WO 2008/006607 A2 discloses pharmaceutical and nutraceutical products comprising vitamin K2, preferably in combination with one or more polyunsaturated fatty acids, which are useful in the treatment or prophylaxis of disorders of the cardiovascular system. This patent application differs from the present invention *inter alia* in that the claimed effects of vitamin K regard prevention of vascular calcification, whereas it is silent on reducing thrombosis risk by increased vitamin K intake.

There are several ways in which thrombosis tendency or increased thrombosis risk may be established, but most of them are based on one of the two following principles:
a) Citrated plasma is collected by venipuncture and clotting is induced by adding tissue factor (thromboplastin) and recalcification. The time required to clot is compared to the clotting time of a reference sample and expressed as International Normalized Ratio (INR). This method is sensitive for monitoring patients during anticoagulation treatment (INR > 2.0), but insensitive for INR values < 1.0 (increased thrombosis risk).
b) Citrated plasma is collected by venipuncture, defibrinated and the conversion of prothrombin into thrombin is initiated by adding tissue factor and calcium. The total amount of thrombin generated in a certain time period is expressed as Endogenous Thrombin Potential (ETP), whereas also other kinetic characteristics (thrombin peak height, time to peak) provide additional information (E. De Smedt, R. Al Dieri, H.M. Spronk, K. Hamulyak, H. ten Cate, H.C. Hemker: Thromb. Haemost. 2008; 100: 343-349)*.*

At this time ETP is the most sensitive biomarker for thrombosis risk, for instance during pregnancy, oral contraceptive use or congenital factor V_{Leiden}. In particular also patients with atrial fibrillation, patients after surgery, those needing bedrest, or subjects undertaking long-distance air flight travelling are known to be at increased thrombosis risk, but in these cases thrombosis risk is caused by other risk factors such as irregular blood flow or stasis of the blood and may not be reflected by elevated ETP values. Nevertheless, also in case of normal ETP and normal vitamin K status, extra vitamin K intake may decrease thrombosis risk via improved protein S carboxylation. And also in this case the effect of vitamin K intervention may be monitored by ETP.

### DETAILED DESCRIPTION OF THE INVENTION

The invention is defined by the claims. Any subject-matter falling outside the scope of the claims is provided for information purposes only. Any references in the description to methods of treatment refer to the compounds, pharmaceutical compositions and medicaments of the present invention for use in a method for treatment of the human or animal body by therapy.

The triage theory posits that, when the availability of a micronutrient is inadequate, nature ensures that micronutrient-dependent functions required for short-term survival are protected at the expense of functions whose lack has only longer-term consequences, such as diseases associated with aging *(*J.C. McCann, B. Ames: Am. J. Clin. Nutr. 2009;90:889-907*).* Without doubt, bleeding is the most lethal short-term consequence of vitamin K deficiency. Consequently, mechanisms have developed during evolution resulting in preferential maturation of blood clotting factors, all synthesized in the liver. Since an adequate supply of vitamin K is vital to clotting factor maturation, it is understandable that the pharmacokinetics of vitamin K result in preferential supply of the liver. Following intestinal absorption, all forms of vitamin K are incorporated into triglyceride-rich lipoproteins which are readily taken up from the blood stream by the liver. Under conditions of poor vitamin K supply, the liver is capable of utilizing all forms of vitamin K for the synthesis of the clotting factors II, VII, IX and X. At adequate vitamin K status, vitamin K2 (notably the long chain menaquinones) is incorporated into low density lipoproteins, released by the liver into the blood stream, and taken up by extra-hepatic tissues known to have LDL receptors (L.J. Schurgers, C. Vermeer: Biochim. Biophys. Acta 2002;1570: 27-32). In this way also extra-hepatic tissues are provided with vitamin K. Obviously, vitamin K-insufficiency will sooner be detected from the occurrence of incompletely carboxylated extra-hepatic Gla-proteins than from uncarboxylated clotting factors. The present invention is based on the hypothesis that - like MGP - also vascular protein S is produced as a mixture of carboxylated and uncarboxylated or partly carboxylated species. If in analogy to MGP also vascular protein S is carboxylated for 70-80%, the anticoagulant activity of the total pool of circulating protein S will be 90% of maximal in healthy volunteers and 70-80% in subjects with a poor vitamin K status, such as chronic kidney disease patients.

In contrast to what is generally thought, we have surprisingly found that mild vitamin K insufficiency may be associated with an increased venous thrombosis risk (e.g. elevated ETP), and that this risk can be decreased by increasing vitamin K intake. This observation was made in the general, healthy population but was even more obvious in patients known to have a poor vitamin K status including but not limited to chronic kidney disease patients.

The present invention thus aims at optimizing the natural blood clotting inhibitory activity. Normal blood clotting activity is generally understood as the pathway from activation of clotting factor VII or IX down the cascade to thrombin generation following the formation of a fibrin clot. Clotting inhibitory activity is the activation of the protein C and protein S system by which factors V and VIII are inactivated. This is not coagulation (clotting, procoagulant activity) but coagulation inhibition (clotting inhibition, anticoagulant activity). The inventors have surprisingly found that clotting inhibition can be considerably improved by administration of vitamin K in addition to dietary intake.

In particular, the present invention relates to vitamin K for use in a method for preventing, decreasing and/or counteracting venous thrombosis risk, preferably increased venous thrombosis risk, in mammalian subjects, preferably human subjects.

In certain embodiments, the invention relates to vitamin K for use in a method for preventing, decreasing and/or counteracting venous thrombosis risk, preferably increased venous thrombosis risk, in mammalian subjects, preferably human subjects, by substantially fully carboxylating the protein S system. According to the invention, a "substantially full" carboxylation of the protein S system is achieved if more than 90%, preferably 95% or more (i.e. ≥ 95%), ≥ 98%, ≥ 99%, ≥ 99.5%, or ≥ 99,9% of circulating protein S is carboxylated.

The form of vitamin K used for preventing, decreasing and/or counteracting venous thrombosis risk is either phylloquinone (vitamin K1) or menaquinone. Compared to phylloquinone, menaquinones are more prone to improve the vascular vitamin K status. Thus, the active ingredient for use according to the invention is preferably selected from one of the menaquinones (vitamin K2) and combinations thereof, and most preferably it is selected from the long-chain menaquinones MK-7, MK-8, MK-9 or MK-10. In certain embodiments of the invention, the vitamin K form used is MK-7. Also encompassed are any combinations of the long-chain menaquinones MK-7, MK-8, MK-9 or MK-10.

According to some embodiments of the invention, vitamin K, in particular at least one menaquinone, may be prepared in the form of a concentrate. Typical examples of this approach are (i) the preparation of vitamin K by organic synthesis, followed by standard purification techniques including chromatography and crystallization and (ii) microbial production, e.g. deep tank fermentation, which is known in the art. These vitamin K products, in particular menaquinone products, have the advantage that they have a controlled constant quality, can be obtained at reasonable costs and can easily be incorporated in pharmaceutical or nutraceutical products without negatively affecting the taste.

Products resulting from organic synthesis may be used in a pure form, wherein "pure" means that the isolation product contains ≥ 80%, preferably ≥ 90%, ≥ 95%, ≥ 98% or ≥ 99.5 % by weight phylloquinone, menaquinone(s) or mixtures thereof and, consequently, ≤ 20% preferably ≤ 10%, ≤ 5%, ≤ 2%, or ≤ 0.5% by weight of other constituents.

Products resulting from microbial fermentation may be used in a pure form or a partially purified form, wherein partially purified means vitamin K concentrations ranging between 0.1% to 20% (w/w). The vitamin K concentrates may be used as such or added to a pharmaceutical or nutraceutical formulation, e.g. those described herein. Further, they may be used for fortifying food products.

According to another embodiment of the invention, the endogenous vitamin K content of foods may be increased, for instance in the production of cheese. Starter ferments for cheese production often contain lactic acid or propionic acid bacteria, which are known to produce menaquinones. By selecting strains with a high menaquinone production, or by creating conditions during cheese preparation and ripening that favor the bacterial synthesis of menaquinones, the vitamin K content of foods may be substantially increased without the need of fortification.

Vitamin K for use according to the invention will preferably be administered in addition to the normal dietary intake of vitamin K. Depending on the normal dietary intake of a given subject and the vitamin K status of this subject before treatment (i.e. at baseline), the dose of vitamin K to be administered according to the invention to achieve the desired effect of preventing, decreasing and/or counteracting venous thrombosis risk, particularly increased venous thrombosis risk, in other words the "effective amount" of vitamin K, will vary within certain limits. Typically, an effect will be seen when administering an amount of vitamin K, preferably menaquinone(s), in the range of between 5 and 10000 µg/day, preferably between and 25 and 2000 µg/day, more preferably between 50 and 1000 µg/day, and most preferably between 100-500 pg/day.

In accordance with preferred embodiments of the invention, the presence of a mildly increased venous thrombosis risk is defined by an Endogenous Thrombin Potential (ETP) between 1 and 2 standard deviations above the average of healthy young adults, and the presence of a pronouncedly increased venous thrombosis risk is defined by an ETP of at least 2 standard deviations above the average of healthy young adults. A definition based on the average of the healthy population and standard deviations is common scientific practice also for other medical conditions, for example in osteoporosis and osteopenia.

Also in case of a normal ETP, the skilled clinician can diagnose an increased venous thrombosis risk, for instance in subjects with recurrent atrial fibrillation or long term bed rest. If the ucOC/tOC ratio in these subjects is > 0, increased vitamin K intake according to the invention will be a valid strategy to decrease the venous thrombosis risk.

"Healthy young adults" within the present invention means men and women between 25 and 45 years of age, not using medication, oral contraceptives or food supplements and no diagnosed congenital or acquired disease, body mass index between 20 and 25, Caucasian race, and with a blood lipid profile (LDL, HDL, cholesterol) within the normal range.

The skilled person will appreciate that precise ETP values depend on reagents and conditions applied in the assay. In some exemplary embodiments, using the reagents and conditions as described in this specification, the average ETP in the normal healthy population is 1800 nM·min, with a standard deviation (SD) of about 400 nM·min. A mildly increased venous thrombosis risk, is then defined at ETP values between 2200 nM·min and < 2600 nM·min, and a more pronouncedly increased venous thrombosis risk, at ETP values ≥ 2600 nM·min.

The ETP reflects the amount of thrombin that can be formed in a period of time following activation of platelet-poor plasma. The amount of thrombin generated is monitored using a chromogenic substrate for thrombin, and results from three independent principles: the circulating concentration of coagulation factors, the concentration of coagulation inhibitors (proteins C and S) and the concentration of thrombin inhibitors such as antithrombin III. Typical ETP curves show a rapid increase of thrombin formed in the first 6-8 minutes after activation, followed by a decline caused by thrombin inhibitors such as antithrombin III. After 30-35 minutes all thrombin has been cleared from the sample. The rate of thrombin generation in the initial phase depends on the concentration and activity of coagulation and anticoagulation factors, whereas the decline in the second phase largely depends on the concentration of antithrombin III. Generally, the ETP is expressed as the area under the curve during the first 30 minutes of thrombin formation.

In a further aspect of the present invention, vitamin K is for use in a method for preventing and/or decreasing venous thrombosis risk, wherein the ETP is maintained below a threshold value of 1 standard deviation above the average of healthy young adults during the entire period of vitamin K treatment.

As already set forth above, the skilled person will appreciate that precise values depend on reagents and conditions applied in the assay. In some embodiments, an exemplary ETP threshold value is 2200 nM·min. Suitable periods of vitamin K treatment are e.g. 2 weeks, 1 month, 3 months, 6 months, 9 months, 1 year, 2 years, 3 years and up to 5 years.

In another aspect of the present invention, Vitamin K is for use in a method for counteracting venous thrombosis risk, wherein the ETP value is reduced by at least 10%, preferably by at least 20% of the subject's baseline value. The baseline ETP value of any given subject may be determined for example according to the methods described herein.

In yet a further aspect, vitamin K used according to the present invention is to be admistered to specific groups of subjects. As already stated *supra*, mammalian subjects according to the invention are in particular human subjects. Vitamin K may, in the context of the invention, be administered to healthy or apparently healthy human subjects, preferably healthy or apparently healthy adult human subjects, which may benefit from vitamin K supplementation. In particular, these subjects are characterized in that they have a regular vitamin K status and a normal thrombosis risk, particularly a normal venous thrombosis risk. The vitamin K status may e.g. be determined using the ucOC/tOC ratio, i.e. the amount of circulating uncarboxylated osteocalcin (ucOC) relative to the total amount of osteocalcin (tOC). The vitamin K status may also be determined using other methods, e.g. the carboxylation degree of the vascular Gla-protein MGP, but since this assay is not yet commercially available, the ucOC/tOC ratio will be used throughout this document.

Thus, healthy or apparently healthy subjects according to the invention are in particular those having
(i) a normal ratio of circulating uncarboxylated to total osteocalcin (ucOC/tOC) and
(ii) a normal ETP value.

Even in healthy or apparently healthy human subjects, there will very often be a certain amount of uncarboxylated circulating osteocalcin. Thus, a "normal ucOC/tOC ratio" in the context of the invention will typically be > 0. More particularly, a "normal ucOC/tOC ratio" in the context of the invention is an ucOC/tOC ratio ≤ 0.125, more particularly > 0 and ≤ 0.125.

A "normal ETP value" in the context of the invention is particularly one that is not higher than 10% above the average standard ETP value of healthy adult control subjects, more particularly an ETP value of ≤ 1980 nM·min.

A further specific group of subjects to which vitamin K may be administered according to the invention are human subjects characterized in that they have a normal vitamin K status, but an elevated thrombosis risk, particularly an elevated venous thrombosis risk. More particularly, those subjects have either
(a)
   (i) a normal ratio of circulating uncarboxylated to total osteocalcin (ucOC/tOC) and
   (ii) a normal ETP value;
or (b)
   (i) a normal ratio of circulating uncarboxylated to total osteocalcin (ucOC/tOC), in particular an ucOC/tOC ratio ≤ 0.125; and
   (ii) an elevated ETP value.

As already mentioned above, in certain conditions associated with an increased thrombosis risk, ETP may have a normal value, and the thombosis risk is increased e.g. because of the altered blood flow. Nonetheless, an increased thrombosis risk in these cases can still be determined by a skilled clinician based on parameters other than the ETP value.

A "elevated ETP value" in the context of the invention is particularly one that is higher than 10% (> 10%) above the average standard ETP value of healthy control subjects, more particularly an ETP value of >1980 nM·min.

Suitable average standard ETP values may e.g. be obtained from a pool of blood or plasma samples from healthy test subjects, which in particular do not suffer from a disease or condition known to be associated with high thrombosis risk and which do not belong to one of the known high thrombosis risk groups, in particular pregnant women, estrogen users (e.g. for birth control), regular smokers, and transplant recipients.

Yet a further specific group of subjects to which vitamin K may be administered according to the invention are human subjects characterized in that they have a poor vitamin K status and a normal thrombosis risk or an elevated thrombosis risk, particularly venous thrombosis risk, not manifesting itself in an elevated ETP value. More particularly, those subjects have
(i) an above average ratio of circulating uncarboxylated to total osteocalcin (ucOC/tOC)
(ii) a normal ETP value.

In the context of the invention, subjects are diagnosed to have a "low" or "poor" vitamin K status in particular if an above average ucOC/tOC ratio is measured. More particularly, an "above average ucOC/tOC ratio" in the context of the invention is an ucOC/tOC ratio > 0.125.

Yet a further specific group of subjects to which vitamin K may be administered according to the invention are human subjects characterized in that they have a poor vitamin K status and an elevated thrombosis risk, particularly an elevated venous thrombosis risk. More particularly, those subjects have
(i) an above average ratio of uncarboxylated to total osteocalcin (ucOC/tOC);
(ii) an elevated ETP value. In a preferred embodiment, these subjects have an ucOC/tOC ratio of > 0.125 and an ETP value of higher than 10% above the average standard ETP value of healthy adult control subjects, more particularly an ETP value of > 1980 nM·min.

Surprisingly, by administration of vitamin K to these groups of subjects in accordance with the invention, in particular in doses > 10 micrograms per day (µg/d), more particularly > 100 µg/d a significant reduction in the ETP value, and thus in the thrombosis risk can be achieved. The effect is most pronounced in subjects with both a poor vitamin K status and an elevated thrombosis risk at baseline.

Subjects with a poor vitamin K status according to the invention are in particular human subjects suffering from a disease selected from the group consisting of gastrointestinal disease, malabsorption, bile obstruction, chronic kidney disease, coronary artery calcification, peripheral artery disease, atherosclerosis, arteriosclerosis, calcifylaxis, atrial fibrillation, and/or from a condition requiring long-term bedrest.

Exemplary conditions where long-term bed rest, i.e. bed rest for at least seven days, preferably at least two weeks, is commonly prescribed are acute pain in the spine or joints; maternal or fetal complications of pregnancy, such as preterm labor, high blood pressure, incompetent cervix, or fetal growth problems, twin pregnancy; cardiac diseases; and acute gout.

Subjects with an increased or elevated venous thrombosis risk, according to the invention are in particular human subjects selected from pregnant women, estrogen users, smokers, patients after surgery, transplant recipients such as renal transplant recipients, patients requiring long-term bedrest, and/or patients suffering from Crohn's disease, congenital thrombophilia (e.g. congenital factor V_{Leiden}), bile obstruction, atrial fibrillation and/or chronic kidney disease. As noted above, an increased venous thrombosis risk can be diagnosed by a skilled clinician even in subjects with a normal ETP value, for instance in subjects with recurrent atrial fibrillation or long-term bed rest. If the ucOC/tOC ratio in these subjects is > 0, increased vitamin K intake according to the invention will be a valid strategy to decrease the venous thrombosis risk.

In a particular embodiment of the invention, the patients to be treated by vitamin K administration are renal transplant recipients.

Also disclosed are patients who have already developed a thrombus or embolism and who are treated with anti-thrombotics other than coumarin derivatives (for instance the direct thrombin or Xa inhibitors) and who may also be given supplemental vitamin K. This has the effect of preventing extension of the thrombus and/or formation of a second thrombus.

In some preferred embodiments of the invention, vitamin K is contained in a pharmaceutical or nutraceutical composition. Pharmaceutical compositions will, in addition to vitamin K as an active ingredient, typically also contain at least one pharmaceutically acceptable carrier, diluent, or excipient. A "pharmaceutically acceptable" carrier, diluent or excipient is typically a compound or mixture of compounds which is physiologically acceptable (e.g. has a physiologically acceptable pH) while retaining the therapeutic properties of the substance with which it is administered. Such carriers, diluents and excipients are well known to the skilled person.

A "nutraceutical" is commonly defined as any substance that may be considered a food or part of a food and provides medical or health benefits, including the prevention and treatment of disease. Such products may range from isolated nutrients, dietary supplements and diets to genetically engineered "designer" foods, herbal products and processed foods such as cereals, soups and beverages.

In accordance with a further aspect of the present invention, the use of vitamin K is provided in the preparation of a food supplement, fortified food, nutraceutical or pharmaceutical product for decreasing or preventing venous thrombosis risk, especially in subjects known to be at risk for thrombosis including pregnant women, estrogen users (both for birth control and postmenopausal complaints), cigarette smokers, patients with Crohn's disease, bile obstruction or congenital factor V_{Leiden}, chronic kidney disease or renal transplant recipients.

In one aspect of the invention, vitamin K or the vitamin K-containing pharmaceutical or nutraceutical composition is free of any further pharmaceutically and/or nutraceutically active ingredients. In one embodiment, the composition may contain further ingredients, but is free of polyunsaturated fatty acids.

In the context of the invention, "free of" means containing less than 0.1% by weight, preferably less than 0.01% by weight, more preferably no detectable amount of the respective compound(s).

In another aspect of the present invention, said vitamin K-containing nutraceutical products may further comprise other compounds known to counteract or prevent elevated thrombosis risk. Examples are so-called "heart-healthy ingredients" such as omega-3 fatty acids, either in pure form or as constituent of marine or vegetable oils (e.g. fish oil, krill oil or walnut oil), vitamins D and B12, and nicotinic acid. Further examples are flavonoids, stanols, sterols, phytosterols, fiber, betaglucan, carotenoids, coenzyme Q10, vitamin E, polyphenols and resveratrol. Also envisaged is the addition of pharmaceutic compounds such as low-dose aspirin.

In particular, such nutraceutical compositions may, in addition to vitamin K, comprise at least one heart-healthy ingredient such as omega-3 fatty acids, vitamin D, vitamin B12, and/or nicotinic acid.

In a further aspect of the present invention, said vitamin K-containing pharmaceutical products may further comprise other medicines known to counteract or prevent elevated thrombosis risk such as platelet aggregation inhibitors (e.g.: clopidogrel, ticlopidin, aspirin, cilostazol, dipyridamole, terutraban), heparin and related substances (e.g.: LMW heparin, synthetic pentasaccharide Xa inhibitors), and the new generation of oral anticoagulants (e.g.: direct thrombin inhibitors such as dabigatran and argatroban; or direct factor Xa inhibitors such as rivaroxaban and apixaban). Furthermore, vitamin K may be combined with cholesterol-lowering drugs such as statins, nicotinic acid, bile acid resins and fibrates.

In particular, such pharmaceutical compositions may, in addition to vitamin K, comprise at least one pharmaceutically active agent known to have a favorable outcome on cardiovascular disease such as aspirin, ACE inhibitors, angiotensin receptor antagonists, beta blockers, statins, direct thrombin inhibitors, factor Xa inhibitors and/or heparin-related anticoagulants.

Only in the case of coumarin-based oral anticoagulant treatment (using vitamin K-antagonists such as warfarin, acenocoumarol, phenprocoumon) the use of vitamin K supplements is not regarded as meaningful or even contra-indicated.

It is also encompassed by the invention that the pharmaceutical or nutraceutical composition in addition to vitamin K may comprise at least one heart-healthy food or food product, e.g. omega-3 fatty acids, vitamin D, vitamin B12, and/or nicotinic acid, and at least one pharmaceutically active agent known to have a favorable outcome on cardiovascular disease, e.g. aspirin, ACE inhibitors, angiotensin receptor antagonists, beta blockers, statins, direct thrombin inhibitors, factor Xa inhibitors and/or heparin-related anticoagulants.

The use of vitamin K according to the invention, in combination with another active substance may take place simultaneously or consecutively, but particularly within a short interval. If they are administered simultaneously, the two active substances are given to the subject together; if they are administereded consecutively, the two active substances are given to the patient within a period of less than or equal to e.g. 12 hours.

Suitable daily doses of vitamin K, in particular of menaquinone to be used in the present invention are in the range between 5 and 5000 micrograms per day, preferably between 25 and 1000 micrograms per day, more preferably between 50 and 500 micrograms and most preferably between 100 and 250 micrograms per day. For patients with a known poor vitamin K status (determined e.g. by the ucOC/tOC ratio) including those with gastrointestinal disease, food malabsorption, bile obstruction, chronic kidney disease, coronary artery calcification, atherosclerosis, arteriosclerosis, calcifylaxis, or peripheral artery disease, these ranges are preferably twice as high, i.e. in the range of between 10 and 10 000 pg/day, preferably between and 50 and 2 000 pg/day, more preferably between 100 and 1 000 pg/day, and most preferably between 200-500 pg/day.

In a further aspect, the invention relates to a method of preventing, decreasing and/or counteracting venous thrombosis risk, preferably increased venous thrombosis risk, wherein said method comprises the step of administering an effective amount of vitamin K, preferably menaquinone, more preferably MK-7, MK-8, MK-9 and/or MK-10, to a mammalian subject, preferably a human subject.

### Formulation of products and dosages

If protected from light, all forms of vitamin K are stable at room temperature with no significant loss after 2 years of storage. The route of administration is preferably systemic (e.g. orally or parenterally), most preferably in the form of capsules, tablets or fortified foods. Since it is a fat-soluble vitamin, it is preferably formulated in oil, for instance fish oil or sunflower seed oil and manufactured in the form of soft or hard gelatin capsules. Vitamin K can also be formulated in tablets comprising pharmaceutically acceptable inactive ingredients such as starch, cellulose, magnesium stearate or mixtures thereof. These products can be prepared by conventional manners known in the art and typically have a weight between 100 milligrams and 1 gram. Moreover, it is well known that through nano-encapsulation fat-soluble vitamins including vitamin K can be prepared in a water-soluble form. The dose of vitamin K per capsule or tablet is typically between 5 and 500 micrograms, preferably between 25 and 250 micrograms, and most preferably between 50 and 250 micrograms. The dose required to significantly decrease a subject's thrombosis risk can be assessed by measuring ETP on a monthly basis during the first months of treatment. Additional biomarkers to be used are desphospho-uncarboxylated matrix Gla-protein (dp-ucMGP, a circulating marker for vascular vitamin K status) and the ucOC/tOC ratio (a widely accepted biomarker for extra-hepatic vitamin K status).

If used as a food supplement or fortified food, formulations may also contain other heart-healthy ingredients including vitamins D and B12, purified Ω-3 fatty acids, marine oils and the like.

If used as a pharmaceutical preparation, formulations may also contain other drugs used in the cardiovascular area, for example ACE inhibitors, angiotensin receptor antagonists, beta blockers, statins and the like. These additives may be combined with vitamin K by preparing a combination product (all in one), but can also be manufactured in separate formulations.

### BRIEF DESCRIPTION OF THE FIGURES

Figure 1 shows the coagulation cascade (A) and the protein C system (B).
Figure 2 represents the endogenous thrombin potential (ETP) with and without excess of APC in a healthy non-supplemented subject with adequate K status (A) and in a similar subject with poor vitamin K status (B).
Figure 3 represents the ETP in the same subjects after supplementing with vitamin K2. (A): subject with adequate vitamin K status at baseline; (B): subject with poor vitamin K status at baseline.
Figure 4 represents the vitamin K1-induced decrease of ETP in healthy subjects as a function of their baseline vitamin K status.

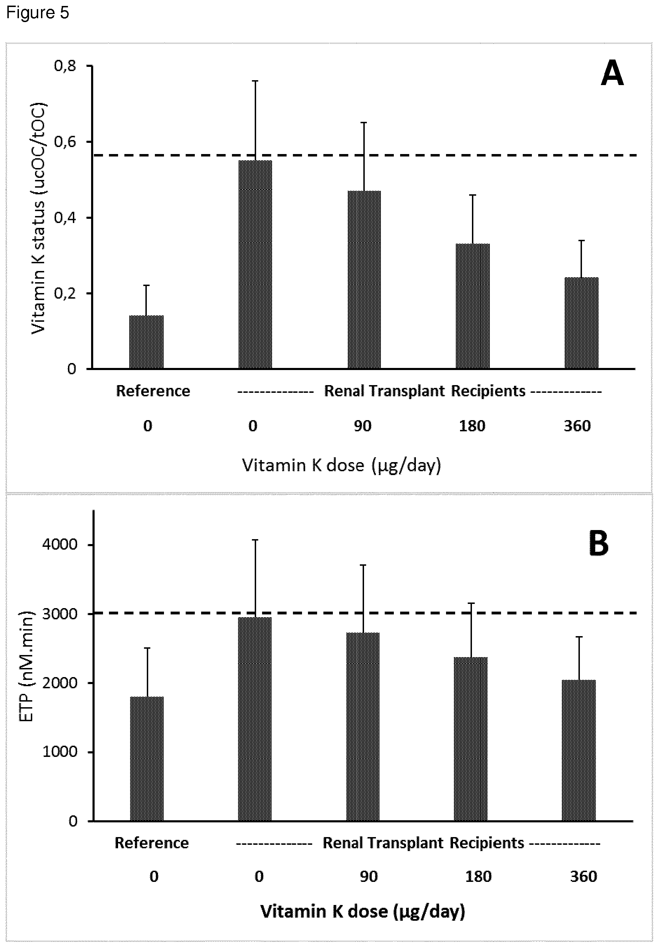

Figure 5 depicts the results of measurements effects of vitamin K2 on vitamin K status and ETP in a dose-dependent way. Figure 5A shows the vitamin K status (expressed as ucOC/tOC) in in blood plasma from a normal reference population and from 32 renal transplant recipients (RTR) between 1 and 2 years after transplantation. The RTR group was divided into 4 groups of 8 subjects and treated with increasing doses of vitamin K2 (menaquinone-7) for 3 months. Figure 5B shows the ETP in the same samples as described in figure 5A. Error bars represent SD, the dotted horizontal lines give the average baseline level of the total group of RTR subjects.

The invention will be further illustrated by the following examples.

### EXAMPLES

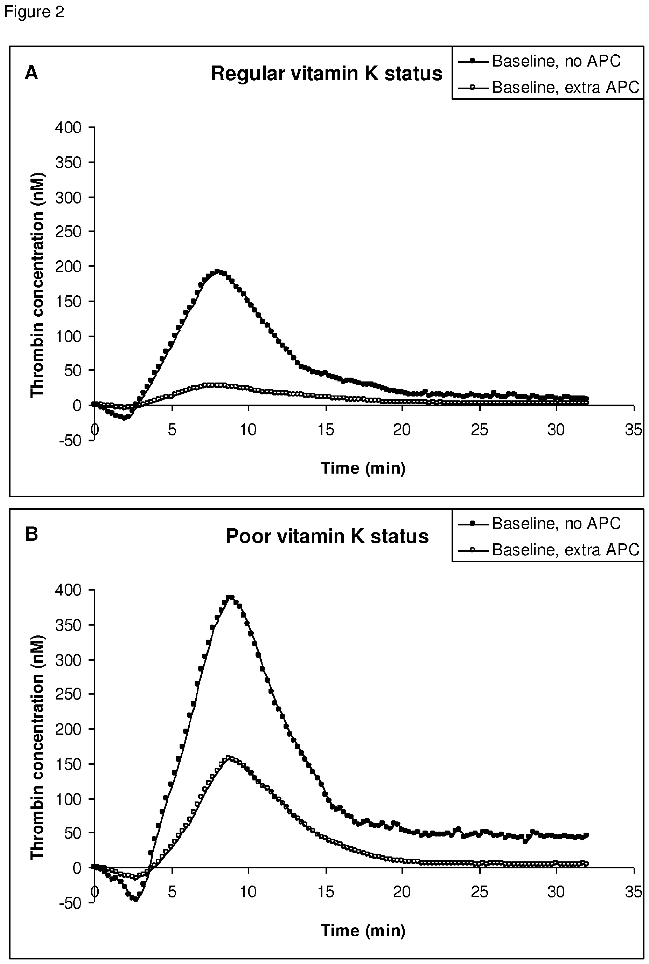

Experiment 1. The vitamin K-status in 242 healthy postmenopausal women was measured on the basis of the ratio between uncarboxylated and carboxylated serum osteocalcin (ucOC/cOC ratio). From this group we selected the 30 subjects with the highest and the 30 subjects with the lowest ucOC/cOC ratio (highest and lowest vitamin K status, respectively) for measuring the ETP. Representative examples from both groups are given in Figure 2A and B (closed symbols). Surprisingly we found that in the group with the highest vitamin K status the ETP (i.e.: thrombosis risk) was significantly lower than that in the group with the lowest vitamin K status. If supplemented with an excess of activated protein C (APC, to induce maximal thrombin inhibition) both curves decreased significantly (Figure 2, open symbols), but in the group with poor vitamin K status the remaining area under the curve was 40% rather than 10% in the group with highest vitamin K status. This is consistent with insufficient active (incompletely carboxylated) protein S in the low-vitamin K group.

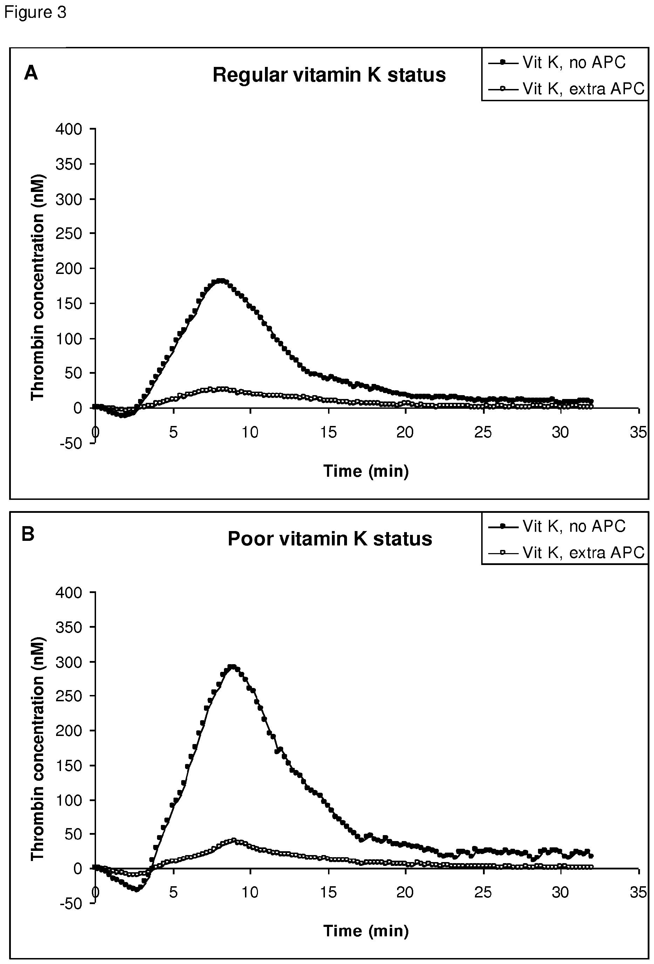

Experiment 2. Subsequently, 15 subjects from each group were treated with a high dose of vitamin K (MK-4, 45 mg/day) during three months, whereas the other 15 received a placebo. In both vitamin K-treated groups the ucOC/cOC ratio declined to virtually zero, indicating vitamin K sufficiency of the extra-hepatic tissues. The ucOC/cOC ratio in the placebo groups remained unchanged. In the group with a low ucOC/cOC ratio at baseline, vitamin K treatment resulted in a non-significant decline of the ETP both before and after adding APC. Figure 3A shows the ETP curves from the same subject as in Figure 2A, but now after 3 months of vitamin K treatment. In the group with a high ucOC/cOC ratio at baseline (poor vitamin K status), not only the average ETP dropped by 25%, also the ETP in the presence of excess APC was normalized to an average of 12.5% of the curves obtained in the absence of APC. Figure 3B shows the ETP curves from the same subject as in Figure 2B, but now after 3 months of vitamin K treatment. This demonstrates that vitamin K supplementation may decrease the thrombosis risk, as measured by ETP. The effect is stronger in subjects having a low vitamin K status at baseline than in those with a high vitamin K status.

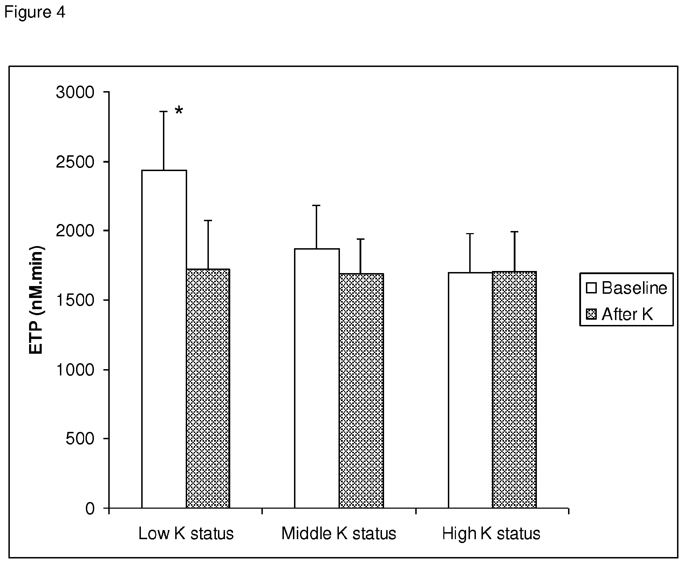

Experiment 3. On the basis of the ratio between uncarboxylated osteocalcin and total osteocalcin (ucOC/tOC ratio), 56 postmenopausal women were selected from a larger cohort and subdivided into three groups: lowest tertile for vitamin K status (ucOC/tOC ratio = 0.20, n=19), middle tertile for vitamin K status (ucOC/tOC ratio = 0.125, n=19) and highest tertile for vitamin K status (ucOC/tOC ratio = 0.071, n=18). All participants were treated with 1 mg/day of vitamin K1 for one year, and the ETP was measured at baseline and after one year of vitamin K treatment. As is shown in Figure 4, the group with the highest ucOC/tOC ratio had the highest ETP. Only in this group the ETP declined significantly after vitamin K supplementation. After one year of vitamin K treatment the average ETP was similar in all three groups.

The interventions described in experiments 2 and 3 were with a high dose of vitamin K (45 mg/day and 1 mg/day, respectively), which obviously is an excess. These experiments were meant to demonstrate the proof-of-principle, and the involvement of vascular protein S in lowering the ETP.

Experiment 4. The ETP was measured in plasma from 8 successive hemodialysis patients and compared with plasma from 12 healthy adults. It was found that in the healthy subjects the ETP ranged between 1325 and 2680 (nmol/l)·min whereas in the hemodialysis patients the values were significantly higher (range: 2177-3412 (nmol/l)·min).

Experiment 5. Renal transplant recipients (RTR) are generally reported to have a mildly decreased vitamin K status, as determined by e.g. osteocalcin carboxylation and MGP carboxylation. In the present study, a group of 32 RTR were randomly subdivided into 4 groups of 8 subjects each and compared with 24 age- and sex-matched healthy volunteers.

The RTR were treated with the following doses of vitamin K2 (menaquinone-7) for 3 months: 0, 90, 180 and 360 µg/day. Blood was taken at baseline and at the end of the study to prepare EDTA plasma and serum. Vitamin K status was assessed by measuring the ucOC/tOC ratio and the thrombosis tendency was estimated from the ETP values.

From Figure 5A it is clear that the ucOC/tOC ratio was elevated in the RTR subjects (as compared to healthy reference population) and that it decreased in a dose-dependent way by vitamin K supplements. This demonstrates that vitamin K status was improved by relatively low dose vitamin K supplements (in the nutritional range). Figure 5B shows that also the ETP decreased at increasing doses of vitamin K intake. The decline (compared to baseline) of both the ucOC/tOC ratio and the ETP values was statistically significant at vitamin K intakes of 180 and 360 pg/day. Also the difference between the 0 µg/day and the 360 µg/day intake groups was significant at t=3 months for both markers. This experiment demonstrates that doses of vitamin K in a relatively low dose range improve vitamin K status in certain patients and that this supplementation results in a concomitant decrease of the thrombosis tendency or risk.

Altogether these data demonstrate that poor vitamin K status is an independent risk factor for thrombosis as measured by ETP, and that this risk factor can be annihilated by increased vitamin K intake. The dose of vitamin K required will depend on the condition of the subjects, and will be higher for those characterized by poor vitamin K status resulting from diet or disease.

## Claims

1. Vitamin K for use in a method for preventing, decreasing and/or counteracting venous thrombosis risk, preferably increased venous thrombosis risk, in mammalian subjects.

2. Vitamin K for use according to claim 1, wherein said mammalian subjects are human subjects.

3. Vitamin K for use according to claim 1 or 2, wherein said vitamin K is selected from phylloquinone (vitamin K1) and menaquinones (vitamin K2), preferably selected from one of the menaquinones (vitamin K2) and combinations thereof, more preferably from long-chain menaquinones MK-7, MK-8, MK-9, MK-10, and combinations thereof.

4. Vitamin K for use according to any one of claims 1 to 3, wherein said vitamin K is prepared in the form of a concentrate, and/or wherein said vitamin K is administered in addition to the normal dietary intake of vitamin K.

5. Vitamin K for use according to any one of claims 1 to 4, wherein the presence of a mildly increased venous thrombosis risk is defined by an Endogenous Thrombin Potential (ETP) between 1 and 2 standard deviations above the average of healthy young adults, and wherein the presence of a pronouncedly increased venous thrombosis risk is defined by an ETP of at least 2 standard deviations above the average of healthy young adults.

6. Vitamin K for use in a method for preventing and/or decreasing venous thrombosis risk according to any one of claims 1 to 5, wherein the ETP is maintained below a threshold value of 1 standard deviation above the average of healthy young adults during the entire period of vitamin K treatment.

7. Vitamin K for use in a method for counteracting venous thrombosis risk according to any one of claims 1 to 5, wherein the ETP value is reduced by at least 10%, preferably by at least 20% of the subject's baseline value.

8. Vitamin K for use according to any one of claims 1 to 7, wherein said subject has
(i) a normal ratio of circulating uncarboxylated to total osteocalcin (ucOC/tOC), in particular an ucOC/tOC ratio ≤ 0.125;
(ii) a normal ETP value, in particular an ETP value that is not higher than 10% above the average standard ETP value of healthy adult control subjects, more particularly an ETP value of ≤ 1980 nM•min;
and wherein the dosage of vitamin K is preferably in the range of between 5 and 5000 pg/day, between 25 and 1000 pg/day, between 50 and 500 pg/day, or between 100-250 pg/day.

9. Vitamin K for use according to any one of claims 1 to 7, wherein said subject has
(i) a normal ratio of circulating uncarboxylated to total osteocalcin (ucOC/tOC), in particular an ucOC/tOC ratio ≤ 0.125;
(ii) an elevated ETP value, in particular an ETP value that is higher than 10% above the average standard ETP value of healthy adult control subjects, more particularly an ETP value of > 1980 nM·min;
and wherein the dosage of vitamin K is preferably in the range of between 5 and 5000 pg/day, between 25 and 1000 pg/day, between 50 and 500 pg/day, or between 100-250 pg/day.

10. Vitamin K for use according to any one of claims 1 to 7, wherein said subject has
(i) an above average ratio of circulating uncarboxylated to total osteocalcin (ucOC/tOC), in particular an ucOC/tOC ratio > 0.125;
(ii) a normal ETP value, in particular an ETP value that is not higher than 10% above the average standard ETP value of healthy adult control subjects, more particularly an ETP value of ≤ 1980 nM•min;
and wherein the dosage of vitamin K is preferably in the range of between 10 and 10 000 pg/day, between and 50 and 2 000 pg/day, between 100 and 1000 pg/day, or between 200-500 pg/day.

11. Vitamin K for use according to any one of claims 1 to 8, wherein said subject has
(i) an above average ratio of circulating uncarboxylated to total osteocalcin (ucOC/tOC), in particular an ucOC/tOC ratio > 0.125;
(ii) an elevated ETP value, in particular an ETP value that is higher than 10% above the average standard ETP value of healthy adult control subjects, more particularly an ETP value of > 1980 nM•min;
and wherein the dosage of vitamin K is preferably in the range of between 10 and 10 000 pg/day, between and 50 and 2 000 pg/day, between 100 and 1 000 pg/day, or between 200-500 pg/day.

12. Vitamin K for use according to claim 10 or 11, wherein said subject suffers from a disease selected from the group consisting of gastrointestinal disease, food malabsorption, bile obstruction, chronic kidney disease, coronary artery calcification, peripheral artery disease, atherosclerosis, arteriosclerosis, calcifylaxis, atrial fibrillation, and/or from a condition requiring long-term bedrest.

13. Vitamin K for use according to any one of claims 1-12, wherein said subject is a human subject selected from pregnant women, estrogen users, smokers, patients after surgery, renal transplant recipients, patients requiring long-term bedrest, and/or patients suffering from disease, congenital thrombophilia, bile obstruction, atrial fibrillation and/or chronic kidney disease.

14. Pharmaceutical or nutraceutical composition comprising vitamin K for use according to any one of claims 1-13.

15. Vitamin K for use according to any one of claims 1-13 or pharmaceutical or nutraceutical composition for use according to claim 14, wherein said vitamin K or said composition is for administration
(i) in combination with at least one heart-healthy food or food product such as omega-3 fatty acids, vitamin D, vitamin B12, and/or nicotinic acid, and/or
(ii) in combination with at least one pharmaceutically active agent known to have a favourable outcome on cardiovascular disease such as aspirin, ACE inhibitors, angiotensin receptor antagonists, beta blockers, statins, direct thrombin inhibitors, factor Xa inhibitors and/or heparin-related anticoagulants.

## Patentansprüche

1. Vitamin K zur Verwendung in einem Verfahren zum Vorbeugen, Verringern und/oder Entgegenwirken eines venösen Thromboserisikos, bevorzugt eines erhöhten venösen Thromboserisikos, bei Säugetiersubjekten.

2. Vitamin K zur Verwendung nach Anspruch 1, wobei die Säugetiersubjekte menschliche Subjekte sind.

3. Vitamin K zur Verwendung nach Anspruch 1 oder 2, wobei das Vitamin K ausgewählt ist aus Phyllochinon (Vitamin K1) und Menachinonen (Vitamin K2), bevorzugt ausgewählt aus einem der Menachinone (Vitamin K2) und Kombinationen davon, besonders bevorzugt aus langkettigen Menachinonen MK-7, MK-8, MK-9, MK-10 und Kombinationen davon.

4. Vitamin K zur Verwendung nach einem der Ansprüche 1 bis 3, wobei das Vitamin K in Form eines Konzentrats hergestellt wird und/oder wobei das Vitamin K zusätzlich zur normalen diätetischen Aufnahme von Vitamin K verabreicht wird.

5. Vitamin K zur Verwendung nach einem der Ansprüche 1 bis 4, wobei das Vorliegen eines leicht erhöhten venösen Thromboserisikos durch ein endogenes Thrombinpotential (ETP) zwischen 1 und 2 Standardabweichungen über dem Durchschnitt gesunder junger Erwachsener definiert ist, und wobei das Vorliegen eines deutlich erhöhten venösen Thromboserisikos durch ein ETP von mindestens 2 Standardabweichungen über dem Durchschnitt gesunder junger Erwachsener definiert ist.

6. Vitamin K zur Verwendung in einem Verfahren zum Vorbeugen und/oder Verringern eines venösen Thromboserisikos nach einem der Ansprüche 1 bis 5, wobei das ETP während des gesamten Zeitraums der Vitamin-K-Behandlung unter einem Schwellenwert von 1 Standardabweichung über dem Durchschnitt gesunder junger Erwachsener gehalten wird.

7. Vitamin K zur Verwendung in einem Verfahren zum Entgegenwirken eines venösen Thromboserisikos nach einem der Ansprüche 1 bis 5, wobei der ETP-Wert um mindestens 10 %, bevorzugt um mindestens 20 % des Ausgangswertes des Subjekts reduziert wird.

8. Vitamin K zur Verwendung nach einem der Ansprüche 1 bis 7, wobei das Subjekt
(i) ein normales Verhältnis von zirkulierendem uncarboxyliertem zu Gesamtosteocalcin (ucOC/tOC) aufweist, insbesondere ein ucOC/tOC-Verhältnis ≤ 0,125;
(ii) einen normalen ETP-Wert aufweist, insbesondere einen ETP-Wert, der nicht höher als 10 % über dem durchschnittlichen Standard-ETP-Wert gesunder erwachsener Kontrollsubjekte liegt, insbesondere einen ETP-Wert von ≤ 1980 nM·min;
und wobei die Dosierung von Vitamin K bevorzugt im Bereich zwischen 5 und 5000 pg / Tag, zwischen 25 und 1000 pg / Tag, zwischen 50 und 500 pg / Tag, oder zwischen 100-250 pg / Tag liegt.

9. Vitamin K zur Verwendung nach einem der Ansprüche 1 bis 7, wobei das Subjekt
(i) ein normales Verhältnis von zirkulierendem uncarboxyliertem zu Gesamtosteocalcin (ucOC/tOC) aufweist, insbesondere ein ucOC/tOC-Verhältnis ≤ 0,125;
(ii) einen erhöhten ETP-Wert aufweist, insbesondere einen ETP-Wert, der höher als 10 % über dem durchschnittlichen Standard-ETP-Wert gesunder erwachsener Kontrollsubjekte liegt, insbesondere einen ETP-Wert von > 1980 nM·min;
und wobei die Dosierung von Vitamin K bevorzugt im Bereich zwischen 5 und 5000 pg / Tag, zwischen 25 und 1000 pg / Tag, zwischen 50 und 500 pg / Tag, oder zwischen 100-250 pg / Tag liegt.

10. Vitamin K zur Verwendung nach einem der Ansprüche 1 bis 7, wobei das Subjekt
(i) ein überdurchschnittliches Verhältnis von zirkulierendem uncarboxyliertem zu Gesamtosteocalcin (ucOC/tOC) aufweist, insbesondere ein ucOC/tOC-Verhältnis > 0,125;
(ii) einen normalen ETP-Wert aufweist, insbesondere einen ETP-Wert, der nicht höher als 10 % über dem durchschnittlichen Standard-ETP-Wert gesunder erwachsener Kontrollsubjekte liegt, insbesondere einen ETP-Wert von ≤ 1980 nM·min;
und wobei die Dosierung von Vitamin K bevorzugt im Bereich zwischen 10 und 10 000 pg / Tag, zwischen 50 und 2000 pg / Tag, zwischen 100 und 1000 pg / Tag, oder zwischen 200-500 pg / Tag liegt.

11. Vitamin K zur Verwendung nach einem der Ansprüche 1 bis 8, wobei das Subjekt
(i) ein überdurchschnittliches Verhältnis von zirkulierendem uncarboxyliertem zu Gesamtosteocalcin (ucOC/tOC) aufweist, insbesondere ein ucOC/tOC-Verhältnis > 0,125;
(ii) einen erhöhten ETP-Wert aufweist, insbesondere einen ETP-Wert, der höher als 10 % über dem durchschnittlichen Standard-ETP-Wert gesunder erwachsener Kontrollsubjekte liegt, insbesondere einen ETP-Wert von > 1980 nM·min;
und wobei die Dosierung von Vitamin K bevorzugt im Bereich zwischen 10 und 10 000 pg / Tag, zwischen 50 und 2000 pg / Tag, zwischen 100 und 1000 pg / Tag, oder zwischen 200-500 pg / Tag liegt.

12. Vitamin K zur Verwendung nach Anspruch 10 oder 11, wobei das Subjekt an einer Erkrankung leidet, die aus der Gruppe ausgewählt ist, die aus gastrointestinaler Erkrankung, Nahrungsmittelmalabsorption, Gallenobstruktion, chronischer Nierenerkrankung, Koronararterienverkalkung, peripherer Arterienerkrankung, Atherosklerose, Arteriosklerose, Verkalkung, Vorhofflimmern und/oder einem Zustand, der eine langfristige Bettruhe erfordert, besteht.

13. Vitamin K zur Verwendung nach einem der Ansprüche 1-12, wobei das Subjekt ein menschliches Subjekt ist, das aus schwangeren Frauen, Östrogennutzern, Rauchern, Patienten nach einer Operation, Empfängern von Nierentransplantaten, Patienten, die langfristige Bettruhe benötigen, und/oder Patienten ausgewählt ist, die an einer Krankheit, kongenitalen Thrombophilie, Gallenobstruktion, Vorhofflimmern und/oder chronischer Nierenerkrankung leiden.

14. Pharmazeutische oder nutrazeutische Zusammensetzung, die Vitamin K zur Verwendung nach einem der Ansprüche 1-13 umfasst.

15. Vitamin K zur Verwendung nach einem der Ansprüche 1-13 oder pharmazeutische oder nutrazeutische Zusammensetzung zur Verwendung nach Anspruch 14, wobei das Vitamin K oder die Zusammensetzung zur Verabreichung ist
(i) in Kombination mit mindestens einem herzgesunden Lebensmittel oder Lebensmittelprodukt, wie Omega-3-Fettsäuren, Vitamin D, Vitamin B12 und/oder Nikotinsäure, und/oder
(ii) in Kombination mit mindestens einem pharmazeutisch aktiven Agens, von dem bekannt ist, dass es einen günstigen Einfluss auf kardiovaskuläre Erkrankungen hat, wie Aspirin, ACE-Inhibitoren, Angiotensinrezeptorantagonisten, Betablocker, Statine, direkte Thrombininhibitoren, Faktor-Xa-Inhibitoren und/oder heparinverwandte Antikoagulantien.

## Revendications

1. Vitamine K pour une utilisation dans un procédé pour la prévention de, la diminution de et/ou la lutte contre le risque d'une thrombose veineuse, de préférence un risque de thrombose veineuse accru, chez des sujets mammifères.

2. Vitamine K pour une utilisation selon la revendication 1, où lesdits sujets mammifères sont des sujets humains.

3. Vitamine K pour une utilisation selon la revendication 1 ou 2, où ladite vitamine K est choisie parmi la phylloquinone (vitamine K1) et des ménaquinones (vitamine K2), de préférence choisie parmi une des ménaquinones (vitamine K2) et des combinaisons de ceux-ci, plus préférablement parmi les ménaquinones à longue chaîne MK-7, MK-8, MK-9, MK-10 et des combinaisons de ceux-ci.

4. Vitamine K pour une utilisation selon l'une quelconque des revendications 1 à 3, où ladite vitamine K est préparée sous la forme d'un concentré et/ou où ladite vitamine K est administrée en plus de l'apport alimentaire normal de vitamine K.

5. Vitamine K pour une utilisation selon l'une quelconque des revendications 1 à 4, où la présence d'un risque de thrombose veineuse accru de façon modérée est définie par un potentiel de thrombine endogène (ETP) entre 1 et 2 écarts types au-dessus de la moyenne d'adultes jeunes en bonne santé et où la présence d'un risque de thrombose veineuse accru de façon prononcée est définie par un ETP d'au moins 2 écarts types au-dessus de la moyenne d'adultes jeunes en bonne santé.

6. Vitamine K pour une utilisation dans un procédé pour la prévention et/ou la diminution d'un risque de thrombose veineuse selon l'une quelconque des revendications 1 à 5, où le ETP est maintenu en dessous d'une valeur seuil de 1 écart type au-dessus de la moyenne d'adultes jeunes en bonne santé pendant la période entière du traitement par la vitamine K.

7. Vitamine K pour une utilisation dans un procédé pour la lutte contre un risque de thrombose veineuse selon l'une quelconque des revendications 1 à 5, où la valeur de ETP est réduite d'au moins 10%, de préférence d'au moins 20% de la valeur de ligne de base du sujet.

8. Vitamine K pour une utilisation selon l'une quelconque des revendications 1 à 7, où ledit sujet a:
(i) un rapport normal d'ostéocalcine non carboxylée dans la circulation sur totale (ucOC/tOC), en particulier un rapport ucOC/tOC ≤ 0,125;
(ii) une valeur de ETP normale, en particulier une valeur de ETP qui n'est pas supérieure à 10% au-dessus de la valeur de ETP standard moyenne de sujets témoins adultes en bonne santé, plus particulièrement une valeur de ETP ≤ 1980 nM•min;
et où la dose de vitamine K est de préférence dans la plage entre 5 et 5000 pg/jour, entre 25 et 1000 pg/jour, entre 50 et 500 pg/jour ou entre 100-250 pg/jour.

9. Vitamine K pour une utilisation selon l'une quelconque des revendications 1 à 7, où ledit sujet a:
(i) un rapport normal d'ostéocalcine non carboxylée dans la circulation sur totale (ucOC/tOC), en particulier un rapport ucOC/tOC ≤ 0,125;
(ii) une valeur de ETP élevée, en particulier une valeur de ETP qui est supérieure à 10% au-dessus de la valeur de ETP standard moyenne de sujets témoins adultes en bonne santé, plus particulièrement une valeur de ETP > 1980 nM•min;
et où la dose de vitamine K est de préférence dans la plage entre 5 et 5000 pg/jour, entre 25 et 1000 pg/jour, entre 50 et 500 pg/jour ou entre 100 et 250 pg/jour.

10. Vitamine K pour une utilisation selon l'une quelconque des revendications 1 à 7, où ledit sujet a:
(i) un rapport moyen élevé d'ostéocalcine non carboxylée dans la circulation sur totale (ucOC/tOC), en particulier un rapport ucOC/tOC > 0,125;
(ii) une valeur de ETP normale, en particulier une valeur de ETP qui n'est pas supérieure à 10% au-dessus de la valeur de ETP standard moyenne de sujets témoins adultes en bonne santé, plus particulièrement une valeur de ETP ≤ 1980 nM•min;
et où la dose de vitamine K est de préférence dans la plage entre 10 et 10 000 pg/jour, entre 50 et 2 000 pg/jour, entre 100 et 1000 pg/jour ou entre 200-500 pg/jour.

11. Vitamine K pour une utilisation selon l'une quelconque des revendications 1 à 8, où ledit sujet a:
(i) un rapport moyen élevé d'ostéocalcine non carboxylée dans la circulation sur totale (ucOC/tOC), en particulier un rapport ucOC/tOC > 0,125;
(ii) une valeur de ETP élevée, en particulier une valeur de ETP qui est supérieure à 10% au-dessus de la valeur de ETP standard moyenne de sujets témoins adultes en bonne santé, plus particulièrement une valeur de ETP > 1980 nM•min;
et où la dose de vitamine K est de préférence dans la plage entre 10 et 10 000 pg/jour, entre 50 et 2 000 pg/jour, entre 100 et 1000 pg/jour ou entre 200-500 pg/jour.

12. Vitamine K pour une utilisation selon la revendication 10 ou 11, où ledit sujet souffre d'une maladie choisie dans le groupe constitué de: maladie gastro-intestinale, malabsorption alimentaire, obstruction biliaire, maladie rénale chronique, calcification d'artère coronaire, maladie d'artère périphérique, athérosclérose, artériosclérose, calciphylaxie, fibrillation auriculaire et/ou d'un état nécessitant un alitement à long terme.

13. Vitamine K pour une utilisation selon l'une quelconque des revendications 1-12, où ledit sujet est un sujet humain choisi parmi des femmes enceintes, des utilisateurs d'œstrogène, des fumeurs, des patients après une chirurgie, des receveurs de transplantation rénale, des patients nécessitant un alitement à long terme et/ou des patients souffrant d'une maladie, d'une thrombophilie congénitale, d'une obstruction biliaire, d'une fibrillation auriculaire et/ou d'une maladie rénale chronique.

14. Composition pharmaceutique ou nutraceutique comprenant de la vitamine K pour une utilisation selon l'une quelconque des revendications 1-13.

15. Vitamine K pour une utilisation selon l'une quelconque des revendications 1-13 ou composition pharmaceutique ou nutraceutique pour une utilisation selon la revendication 14, où ladite vitamine K ou ladite composition est pour une administration:
(i) en combinaison avec au moins un aliment ou un produit alimentaire bon pour le cœur tel que des acides gras oméga-3, la vitamine D, la vitamine B12 et/ou l'acide nicotinique, et/ou
(ii) en combinaison avec au moins un agent pharmaceutiquement actif connu pour avoir un résultat favorable sur une maladie cardiovasculaire tel que l'aspirine, des inhibiteurs de ACE, des antagonistes de récepteur d'angiotensine, des bêtabloquants, des statines, des inhibiteurs directs de thrombine, des inhibiteurs du facteur Xa et/ou des anticoagulants apparentés à l'héparine.
